# EUROPEAN PATENT APPLICATION

(11) **EP 1 481 688 A1**
(43) Date of publication of application: **01.12.2004**
(21) Application number: 02702730.9
(22) Date of filing: 05.03.2002
(51) Int. Cl.: A61K 39/39, A61K 39/00, A61P 35/00

(54) **SOLIDIFIED TISSUE IMMUNOLOGICAL ADJUVANT**

(71) Applicant: Cell-Medicine, Inc., Ushiku-shi, Ibaraki 300-1234 (JP)
(72) Inventor: OHNO, Tadao, Ushiku-shi, Ibaraki 300-1234 (JP); UCHIMURA, Eiji, Tsukuba-shi, Ibaraki 305-0024 (JP)
(74) Representative: Sternagel, Fleischer, Godemeyer & Partner
(86) International application number: PCT/JP2002/002013
(87) International publication number: WO 2003/074079

(57) **Abstract**

An immunoadjuvant, having a potent cell-stimulating effect and highly safe for living bodies, which comprises a fragment, wherein said fragment is prepared from a solidified material selected from the group consisting of a tissue and a cell of an animal including a human and an ingredient thereof, and from which fragment a soluble ingredient is removed by washing with an organic solvent and/or hot water, and wherein a soluble ingredient derived from a microorganism is immobilized to said fragment.

## Description

### Technical Field

The present invention relates to a method for producing a solidified tissue immunoadjuvant. Said method is useful as a method for strengthening general immunoreactions, as well as useful for prevention of recurrence of tumor, inhibition of metastasis of tumor, and therapeutic treatment of tumor on the basis of antitumor immunoreactions.

### Background Art

Various kinds of conventionally known immunoadjuvants are available which enhance immunoreactions against antigens when administered to living individuals together with antigens. Almost all of the immunoadjuvants cause inflammatory reactions and activate immunocompetent cells gathering in a part affected with inflammatory reaction. In recent years, it has been elucidated that, among immunocompetent cells, those playing a chief role in the immunoreactions are antigen-presenting cells, and the most potent cells among them are dendritic cells (Dentdritic Cells, Second edition, ed. by Lotze, M.T. and Thomson, A.W., Academic Press, San Diego, 2001). It has also been revealed that immature dendritic cells, which can phagocytize microparticle antigens, flow in peripheral blood, and when lipopolysaccharide (LPS) as a main ingredient of endotoxins is added to dendritic cells in vitro, the dendritic cells become mature dendritic cells having potent ability to present antigens. The dendritic cells activated in this process release various kinds of cytokines, such as granulocyte-macrophage colony stimulating factor (GM-CSF), interleukin (IL) 12, and interferon-gamma (IFNg). Moreover, GM-CSF itself is essential as a cell growth factor of the dendritic cells. Therefore, the dendritic cells once activated become possible to maintain the activated state for a long period of time and continue to survive on the basis of the autocline mechanism of GM-CSF.

It is well-known that a direct administration of an endotoxin or *Escherichia coli* containing the same to a living body causes a severe shock symptom. Therefore, relatively safe bacteria that do not have such action (for example, *Mycobacterium bovis* BCG (henceforth referred to as "BCG bacterium")) are used as immunoadjuvants. However, although these bacteria are relatively safe, they often induce undesirable side effects when the bacteria or bacterial components, per se, are used. For example, it is a widely known fact that BCG bacterium cells cause ulcer on a skin to which they are injected. When the cells of *Corynebacterium parvum* (hereinafter abbreviated as *"C. parvum*") are injected to an animal, as a tumor immunity experimental system aiming at a control of a tumor, the cells exhibit a potent anti-tumor action. However, the cells also cause spleen hypertrophy and liver hypertrophy. It is known that ingredients in a pyridine extract obtained from the *C. parvum* cells exhibit an antitumor action without causing the above undesirable reactions, and this phenomenon is observed in the same manner in other anti-tumor reaction-inducing bacteria, such as the BCG bacterium (Cantrell, J.L., US Patent No. 4,663,306). It has been an important object in the field of immunology to develop a tumor immunoadjuvant which maintains an adjuvant activity and has no toxicity to a living body.

Among the variety of conventionally known immunoadjuvants, only a few immunoadjuvants are safe enough to be usable in a tumor immunotherapy for a purpose of therapeutic treatment, metastasis prevention, and recurrence of human tumors, and also are inexpensive. For example, keyhole limpet hemocianin (KLH) has been used as an adjuvant in a tumor immunotherapy by using cultured dendritic cells (Geiger, J.D., et al., Cancer Res., 61:8513-8519, 2001). However, KLH has a problem that the substance is extremely expensive because it is extracted from keyhole limpets. Methods of directly administering cytokines such as GM-CSF, which activate dendritic cells, as adjuvants are also available. However, cytokines are still much more expensive. Moreover, these substances including the pyridine extract from C. parvum cells are soluble, and thus they have a problem that they rapidly diffuse and disappear in a body.

Ingredients in a culture filtrate of *M. tuberculosis* exhibit only a weak adjuvant activity in a dissolved state, whilst they promote T-cell reactions by being bound to polystyrene microparticles (Wilkinson, K.A., et al., J. Immunol. Methods, 235:1-9, 2000). When an adjuvant in a dissolved state is immobilized on an insoluble adjuvant carrier, the adjuvant does not rapidly diffuse and disappear to allow to exhibit a potent adjuvant activity. The polystyrene microparticles are phagocytized by antigen-presenting cells. However, since they are not decomposed in the cells, they will become undesirable plastic residua in a body.

As a method for solving this problem, the above publication also refers to a method of binding the ingredients in the culture filtrate of the bacterium to microparticles consisting of a synthetic biodegradable polymer (Vordermeier, H.M., et al., Vaccine, 13, 1576-1582, 1995; Ertl, H.C., et al., Vaccine, 14, 879-885, 1996; Jones D.H., et al., J. Biotechnology, 44, 29-36, 1996; Venkataprasad, N., et al., Vaccine, 17, 1814-1819, 1999). However, it has been found that the described synthetic polymer, poly(DL-lactide co-glycolide) (PLG) generates lactic acid upon decomposition, and it acidifies an environment of the site of the decomposition. Thus, the method is also not desirable for a living body.

### Disclosure of the Invention

In the field of tumor immunology, an adjuvant carrier has been desired which does not exhibit the undesirable actions as mentioned above, even in a solid state and moreover in biodegradable form. In the state of art, no method is available which comprises the step of binding an immunoadjuvant to a tissue or tumor cells containing complex and diverse tumor antigens, after solidification thereof, and supplying the result as a whole to antigen-presenting cells to efficiently stimulate the tumor antigen disposition by the cells.

The inventors of the present invention conducted various researches to solve the aforementioned problems, and as a result, they found that a superior adjuvant was successfully provided by combining the following ideas:
(1) A tumor tissue solidified and made into microparticles is administered in vivo with a cytokine such as GM-CSF, antitumor immunoreactions against tumor cells can be efficiently induced (PCT/JP00/00692, "Tumor Vaccine"). Therefore, GM-CSF itself serves as an adjuvant, and thus the amount of GM-CSF produced from antigen-presenting cells stimulated by an immunoadjuvant can be served as an index representing the activity of the original adjuvant.
(2) In the process of antitumor immunoreactions, dead tumor cells are phagocytized and decomposed by antigen-presenting cells, and the tumor antigens produced thereby are presented on major histocompatibility complex (MHC) class I molecules on the antigen-presenting cell surfaces to activate cytotoxic T lymphocytes (CTL) which kill tumor cells (Dhodapkar K.M., et al., J. Exp. Med., 195:125-133, 2002).
   Therefore, it is considered that if a substance having an adjuvant activity is stuck by a fixation treatment to a tumor tissue in which tumor cells are killed, the substance having an adjuvant activity and the dead tumor cells will be simultaneously carried in the antigen-presenting cells by the phagocytosis action. In this process, when a fixed and solidified biological tissue is used, which may not necessarily be a tumor tissue, a soluble substance having only a weak adjuvant activity is phagocytized integrally with the biological tissue to which the substance is stuck, and therefore it acts as a solidified insoluble adjuvant and becomes able to most efficiently stimulate phagocytotic cells. In addition, the solidified biological tissue itself, which is used as a carrier, is degraded and disposed in the cells, and therefore the solid product is highly safe as an adjuvant.
(3) Formalin causes a cross-linking reaction between biopolymers, and the reticularly bound biopolymers are insolubilized. The so-called formalin fixation of biological tissues is performed by this reaction. Since the invention of the Salk vaccine for poliomyelitis, formalin-inactivated vaccines have been frequently used. In dental treatments, formalin is regularly used for fixation of dental pulps. This treatment is an operation to kill a nerve in the teeth. Although the most part of the fixed dental pulp is physically removed, the residual fixed tissue remaining in the dental pulp itself has no effect on the host individual, and will disappear with passage of time. These findings suggest that a formalin-fixed biological tissue causes no problem concerning safety. Therefore, if a formalin-fixed tissue is used as a solidified biological tissue, the tissue will be highly safe which can be administered to a human.
(4) It is well known that, in a human, when live cells derived from a different individual are used, the cells exhibit an adjuvant activity due to an immunorejection against the tissues of the different individual (for example, when lymphocytes from different individual are mixed, a potent lymphocyte proliferation is induced). A fixed tissue derived from a different individual may possibly maintain an immunoadjuvant activity, and the tissue may presumably be usable as a general immunoadjuvant. Further, a tissue from an animal other than a human, such as a porcine tissue, induces a potent immunorejection when transplanted to a human. Accordingly, it is expected that even a fixed porcine tissue may have a sufficient adjuvant activity. In human, when an extracted tumor tissue from a tumor patient is fixed and used as an adjuvant carrier, all kinds of tumor antigens contained in the tissue are considered to be taken up into antigen-presenting cells together with the stuck substance having an adjuvant activity.
(5) Furthermore, when a resected tumor tissue of a tumor patient is autologously administered to said tumor patient, substances contained in the tissue, other than tumor antigens, are inherently the same as those in a normal tissue of the patient, and therefore, they are not recognized as antigens. In this process, when a solidified resected tumor tissue is administered with an immunoadjuvant, as an integrated form, they can efficiently stimulate specific immunoreactions only to tumor antigens inherent to the tumor patient, and may serve as a superior autologous tumor vaccine.
(6) For measurement of an adjuvant activity in a human, an amount of GM-CSF produced by blood adhesive cells including dendritic cells is used as an index to carry out the measurement, and the measurement is performable in an in vitro experiment system.

The present invention thus provides an immunoadjuvant comprising a fragment, wherein said fragment is prepared from a solidified material selected from the group consisting of a tissue, a cell, and their components of an animal including a human, and from which fragment a soluble ingredient is removed by washing with an organic solvent and/or hot water, and wherein a soluble ingredient derived from a microorganism is immobilized to said fragment.

From another aspect, the present invention provides a method for producing an immunoadjuvant, which comprises the following steps of:
(a) washing a fragment, which is prepared from a solidified material selected from the group consisting of a tissue and a cell of an animal including a human and an ingredient thereof, with an organic solvent and/or hot water to remove a soluble ingredient, and
(b) immobilizing a soluble ingredient derived from a microorganism to the fragment obtained in the step (a).

According to a preferred embodiment of the aforementioned invention, a tumor tissue and/or a tumor cell is used as the human tissue or cell, and a formalin-fixed tissue and/or a formalin-fixed cell can be used as the solidified material selected from the group consisting of a tissue and a cell of an animal including a human and an ingredient thereof. Further, a bacterium can be used as the microorganism, and as the soluble ingredients derived from a microorganism, an alcohol extract, an acetone extract, a pyridine extract, or a hot water extract can be used.

From a further aspect, the present invention provides a tumor vaccine comprising, as an active ingredient, the aforementioned immunoadjuvant prepared by using a tumor tissue and/or a tumor cell as the human tissue or cell. Furthermore, from still further aspect, the present invention provides a method for therapeutic treatment of a tumor, which comprises the step of administering the aforementioned immunoadjuvant to a patient from whom the tumor is derived.

### Best Mode for Carrying out the Invention

The immunoadjuvant of the present invention is characterized to comprise a fragment which is prepared from a solidified material selected from the group consisting of tissues and cells of animals including human and ingredients thereof (hereinafter in the specification, said material may be occasionally referred to as a "solidified biological material"), from which soluble ingredients are removed by washing with an organic solvent and/or hot water, and to which soluble ingredients derived from a microorganism are immobilized.

The immunoadjuvant of the present invention is an insoluble adjuvant on which soluble ingredients derived from a microorganism having a weak adjuvant activity, preferably soluble ingredients derived from a bacterium, are immobilized, and when the adjuvant is phagocytized by a cell, it gives stimuli from a cell surface at close range or from inside of the cell, and thus act as an adjuvant having a potent activity.

According to a preferred embodiment of the present invention, trace amounts of soluble ingredients, which are extracted from a bacterium or the like and having a weak adjuvant activity, are immobilized on a fragment of the solidified biological material, thereby a release of GM-CSF from blood adhesive cells can be induced at a high concentration which is comparable to that obtainable by stimulation with LPS.

Further, according to another preferred embodiment, the present invention provides the immunoadjuvant prepared by using a formalin-fixed tumor tissue fragment, obtained after a surgical operation of a tumor patient, as the fragment of the solidified biological material and immobilizing on the fragment a trace amount of soluble ingredients having a weak adjuvant activity which are extracted from a microorganism, preferably a bacterium. By allowing the immunoadjuvant to be taken up by antigen-presenting cells, among human blood adhesive cells (monocytes, macrophages, immature dendritic cells and the like), or by antigen-presenting cells in body tissues, CTL that reacts with tumor antigens contained in the tumor tissue can be induced, and the tumor cell-killing action of the CTL enables prevention of recurrence of the cancer, prevention of metastasis, and/or therapeutic treatment of the residual cancer in the postoperative patient.

The method for producing an immunoadjuvant of the present invention typically comprises the following steps:
(a) washing a fragment, which is prepared from a solidified material selected from the group consisting of tissues and cells of animals including human and ingredients thereof, with an organic solvent and/or hot water to remove soluble ingredients, and
(b) extracting soluble ingredients from a microorganism, preferably a bacterium, having an adjuvant activity, with an organic solvent and/or hot water, and then contacting the resulting extract with the fragment obtained in the step (a) to immobilize the soluble ingredients on the fragment.

According to the aforementioned step (b), by washing the fragment of the solidified biological material, on which the soluble ingredients are immobilized, with water or usual physiological saline to remove unadsorbed soluble ingredients, an adjuvant on which soluble ingredients that are not easily released are immobilized, which are among the soluble ingredients derived from the microorganism, can be produced.

A type of the solidified biological material used in the above step (a) is not particularly limited. A biological tissue fixed with formalin, for example, can be used. The biological tissue fixed with formalin, per se, is usually contains a significant amount of lipids. It is desirable that the tissue is degreased by washing with an alcohol or hot water.

Fragmentation of the solidified biological material facilitates the degreasing, and alcohol-soluble ingredients and hot water-soluble ingredients (for example, low molecule ingredients such as peptides) can also be removed in addition to lipids. As a result, a skeletal structure of a biological tissue remains, which is insolubilized by intermolecular cross-links formed by formalin, and this structure is particularly preferred as the solidified biological material.

A method of preparing a fragment from a solidified biological material is not particularly limited. Any methods well known to those skilled in the art, for example, means for preparing small fragments by using a homogenizer, usual means for disruption and the like can be used. In the specification, the term "fragment" means a small preparation prepared by means of disruption, cutting or the like. The means for production is not limited, and the "fragment" should not be construed in any limitative sense. It is generally preferable to prepare a fragment having a size of about 0.04 mm. However, the size of the fragment is not particularly limited.

A method of washing the fragment of the solidified biological material with an organic solvent and/or hot water is not particularly limited. Any methods well known to those skilled in the art may be used. For example, the washing is preferably performed by using ethanol or hot water at a temperature higher than 40°C. A mixture of an organic solvent (preferably ethanol) and hot water may be used for the washing, or washing with an organic solvent and washing with hot water may be performed successively. Such washing may be performed repeatedly. An amount of the organic solvent and/or hot water on the basis of an amount of the fragments of the solidified biological material is not particularly limited. For example, 50 to 100 ml of an organic solvent and/or hot water for 1 g of the solidified biological tissues before the preparation of the fragments may preferably be used. After an organic solvent and/or hot water is added to the fragments and sufficiently stirred, washed fragments can be prepared by centrifuging the mixture and removing a supernatant.

As the microorganism usable in the above step (b), for example, bacteria, fungi, Actinomycetes and the like can be exemplified. Bacteria can be preferably used. Examples of more preferred bacteria are listed below. Any one kind of these bacteria may be used, or two or more kinds of these bacteria may be used in combination. *Corynebacterium diphtheriae*; *Corynebacterium pseudotuberculosis*; *Corynebacterium xerosis*; *Corynebacterium renale*; *Corynebacterium kutscheri; Corynebacterium pseudodiphtheriticum; Corynebacterium equi; Corynebacterium bovis*; *Corynebacterium parvum; Corynebacterium paurometabolum; Corynebacterium pyogenes*; *Corynebacterium enzymicum*; *Corynebacterium hoagii; Corynebacterium striatum; Corynebacterium murisepticum; Corynebacterium nephridii; Corynebacterium phocae*; *Corynebacterium vaginalis*; *Microbacterium flavum*; *Corynebacterium fascians*; *Corynebacterium rathayi; Corynebacterium agropyri*; *Corynebacterium tritici*; *Corynebacterium iranicum; Corynebacterium sepedonicum;* *Corynebacterium beticola; Corynebacterium ilicis*; *Corynebacterium humiferum*; *Corynebacterium humuli*; *Corynebacterium hypertrophicans*; *Corynebacterium acetoacidophilum*; *Corynebacterium acetophilum*; *Corynebacterium* aurantiacum; *Corynbacterium callunae; Corynebacterium citreum-mobilis; Corynebacterium ethanolaminophilum; Corynebacterium flaccumfaciens*; *Corynebacterium glutamicum*; *Corynebacterium herculis*; *Corynebacterium hydrocarboclastus*; *Corynebacterium lilium*; *Corynebacterium luteum; Corynebacterium mediolanum*; *Corynebacterium melassecola*; *Corynebacterium mycetoides*; *Corynebacterium nubilum; Corynebacterium roseum; Corynebacterium sanguinis*; *Arthrobacter globiformis*; *Arthrobacter simplex*; *Arthrobacter tumescens*; *Arthrobacter citreus*; *Arthrobacter terregens*; *Arthrobacter flavescens*; *Arthrobacter duodecadis*; *Arthrobacter luteus*; *Arthrobacter marinus; Arthrobacter variabilis; Arthrobacter viscosus; Arthrobacter polychromogenes; Arthrobacter consociatus; Arthrobacter nicotinovorus; Brevibacterium linens; Brevibacterium acetylicum*; *Brevibacterium erythrogenes*; *Brevibacterium healii*; *Brevibacterium lipolyticum; Brevibacterium brunneum; Brevibacterium fulvum; Brevibacterium fuscum*; *Brevibacterium helvolum*; *Brevibacterium immotum; Brevibacterium marinopiscum; Brevibacterium sociovivum; Brevibacterium stationis*; *Brevibacterium maris*; *Brevibacterium imperiale*; *Brevibacterium incertum*; *Brevibacterium insectiphilium*; *Brevibacterium minutiferula*; *Brevibacterium quale*; *Brevibacterium tegumenticola; Brevibacterium ammoniagenes*; *Brevibacterium sulfureum; Brevibacterium protophormiae*; *Brevibacterium saperdae; Brevibacterium flavum; Brevibacterium immariophilum; Brevibacterium lactofermentum*; *Brevibacterium roseum; Brevibacterium saccharolyticum; Brevibacterium divaricatum*; *Brevibacterium leucinophagum*; *Brevibacterium liquefaciens*; *Brevibacterium pentoso-alanicum*; *Brevibacterium pentoso-aminoacidicum; Brevibacterium lyticum; Brevibacterium albidum*; *Brevibacterium citreum; Brevibacterium luteum; Brevibacterium testaceum; Brevibacterium pusillum*; *Brevibacterium alanicum; Brevibacterium aminogenes*; *Brevibacterium chromogenes*; *Brevibacterium frigoritolerans*; *Brevibacterium halotolerans*; *Brevibacterium fermentans*; *Brevibacterium oxydans*; *Microbacterium lacticum; Microbacterium liquefaciens*; *Microbacterium flavum; Microbacterium thermosphactum; Cellulomonas flavigena; Cellulomonas acidula; Cellulomonas aurogena*; *Cellulomonas galba; Cellulomonas pusilla; Kurthia zopfii; Kurthia* *variabilis*; *Kurthia bessonii; Propionibacterium freudenreichii*; *Propionibacterium thoenii*; *Propionibacterium acidi-propionici*; *Propionibacterium jensenii*; *Propionibacterium avidum; Propionibacterium acnes; Propionibacterium acnes Type II*; *Propionibacterium lymphophilum*; *Propionibacterium granulosum*; *Eucobacterium foedans*; *Eucobacterium alactolyticum*; *Eucobacterium rectale*; *Eucobacterium limosum; Eucobacterium ruminantium; Eucobacterium saburreum; Eucobacterium budayi*; *Eucobacterium nitritogenes*; *Eucobacterium ventriosum; Eucobacterium mutiforme*; *Eucobacterium cylindroids*; *Eucobacterium moniliforme*; *Eucobacterium tortuosum; Eucobacterium cellulosolvens; Eucobacterium combesii; Eucobacterium tenue*; *Eucobacterium fissicatena*; *Eucobacterium contortum; Eucobacterium aerofaciens*; *Eucobacterium lentum*; *Eucobacterium endocarditidis*; *Eucobacterium helminthoides*; *Eucobacterium pseudotortuosum; Eucobacterium obstii; Eucobacterium ethylicum*; *Eucobacterium helwigiae; Eucobacterium ureolyticum*; *Eucobacterium parvum; Actinomyces bovis*; *Actinomyces odontolyticus*; *Actinomyces israelii*; *Actinomyces naeslundii*; *Actinomyces viscosus*; *Actinomyces eriksonii; Actinomyces humiferus*; *Actinomyces suis*; *Arachnia propionica; Bifidobacterium bifidum; Bifidobacterium adolescentis*; *Bifidobacterium infantis*; *Bifidobacterium breve*; *Bifidobacterium longum; Bifidobacterium pseudolongum; Bifidobacterium thermophilum*; *Bifidobacterium suis*; *Bifidobacterium asteroids*; *Bifidobacterium indicum*; *Bifidobacterium coryneforme*; *Bacterionema matruchotii; Rothia dentocariosa*; *Mycobacterium tuberculosis*; *Mycobacterium microti*; *Mycobacterium bovis; Mycobacterium bovis BCG; Mycobacterium africanum; Mycobacterium kansasii; Mycobacterium marinum; Mycobacterium simiae*; *Mycobacterium gastri; Mycobacterium nonchromogenicum*; *Mycobacterium terrae*; *Mycobacterium triviale*; *Mycobacterium gordonae*; *Mycobacterium scrofulaceum*; *Mycobacterium paraffinicum*; *Mycobacterium intracellulare*; *Mycobacterium avium*; *Mycobacterium xenopi*; *Mycobacterium ulcerans*; *Mycobacterium phlei; Mycobacterium vaccae*; *Mycobacterium diernhoferi; Mycobacterium smegmatis; Mycobacterium thamnopheos*; *Mycobacterium flavescens*; *Mycobacterium fortuitum; Mycobacterium peregrinum; Mycobacterium chelonei; Mycobacterium paratuberculosis*; *Mycobacterium leprae*; *Mycobacterium lepraemurium*; *Frankia alni*; *Frankia elaeagni*; *Frankia discariae*; *Frankia ceanothi*; *Frankia coriariae*; *Frankia dryadis*; *Frankia purshiae*; *Frankia cercocarpi*; *Frankia brunchorstii*; *Frankia casuarinae*; *Actinoplanes philippinensis*; *Actinoplanes armeniacus*; *Actinoplanes missouriensis*; *Actinoplanes utahensis*; *Spirillospora albida*; *Streptosporangium roseum; Streptosporangium vulgare*; *Streptosporangium amethystogenes*; *Streptosporangium pseudovulgare*; *Streptosporangium nondiastaticum*; *Streptosporangium longisporum; Streptosporangium viridogriseum*; *Streptosporangium album; Streptosporangium albidum*; *Streptosporangium viridialbum; Streptosporangium rubrum; Amorphosphorangium auranticolor*; *Ampullariella regularis*; *Ampullariella campanulata*; *Ampullariella lobata; Ampullariella digitata; Pilimelia terevasa; Pilimelia anulata*; *Planomonospora parontospora*; *Planomonospora venezuelensis*; *Planobispora longispora; Planobispora rosea; Dactylosporangium aurantiacum*; *Dactylosporangium thailandense*; *Dermatophilus congolensis*; *Geodermatophilus obscurus*; *Nocardia farcinica; Nocardia otitidus-caviarum*; *Nocardia brasiliensis*; *Nocardia asteroids*; *Nocardia transvalensis*; *Nocardia formicae*; *Nocardia coeliaca*; *Nocardia polychromogenes*; *Nocardia paraffinae*; *Nocardia petroleophila*; *Nocardia saturnea; Nocardia kuroishii; Nocardia rugosa; Nocardia rhodnii; Nocardia vaccinii; Nocardia minima; Nocardia blackwellii*; *Nocardia convoluta; Nocardia cellulans*; *Nocardia lutea; Nocardia globerula*; *Nocardia rubropertincta*; *Nocardia corallina; Nocardia salmonicolor*; *Nocardia rubra*; *Nocardia opaca; Nocardia calcarea*; *Nocardia restricta*; *Nocardia erythropolis*; *Nocardia marina; Nocardia atlantica*; *Nocardia aerocolonigenes*; *Nocardia aurantia; Nocardia butanica; Nocardia dassonvillei*; *Nocardia histidans*; *Nocardia madurae*; *Nocardia neoopaca; Nocardia pellegrino*; *Nocardia pelletieri; Nocardia sylvodorifera*; *Nocardia turbata; Nocardia tenuis*; *Nocardia variabilis*; *Pseudonocardia thermophila*; *Pseudonocardia spinosa*; *Streptomyces albolongus*; *Streptomyces viridaris*; *Streptomyces albo-niger, Streptomyces albosporeus*; *Streptomyces albovinaceus*; *Streptomyces aureocirculatus*; *Streptomyces baarnensis*; *Streptomyces clavifer*; *Streptomyces galtieri*; *Streptomyces bobili*; *Streptomyces longispororuber*; *Streptomyces longisporus*; *Streptomyces herbeus*; *Streptomyces albofaciens*; *Streptomyces albus*; *Streptomyces albus* subsp. *bruneomycini*; *Streptomyces albus* subsp. *pathocidicus*; *Streptomyces almquistii*; *Streptomyces aminophilus*; *Streptomyces cacaoi*; *Streptomyces chrestomyceticus*; *Streptomyces flocculus*; *Streptomyces gibsonii; Streptomyces herbescens*; *Streptomyces iodoformicus*; *Streptomyces ochraceiscleroticus*; *Streptomyces rangoon; Streptomyces rimosus*; *Streptomyces rimosus* subsp. *paromomycinus*; *Streptomyces* *rimosus subsp. pseudoverticillatus*; *Streptomyces spiroverticillatus*; *Streptomyces subflavus*; *Streptomyces varsoviensis*; *Streptomyces xantholiticus*; *Streptomyces albus* subsp. *fungatus*; *Streptomyces hydrogenans*; *Streptomyces vendargus*; *Streptomyces achromogenes*; *Streptomyces antibioticus*; *Streptomyces bikiniensis*; *Streptomyces cacaoi* subsp. *asoensis*; *Streptomyces cinereoruber*; *Streptomyces cinereoruber subsp. fructofermentans*; *Streptomyces cylindrosporussubsp. piceus*; *Streptomyces ederensis*; *Streptomyces fulvoviolaceus*; *Streptomyces fulvoviridis*; *Streptomyces gardneri*; *Streptomyces globosus*; *Streptomyces griseorubiginosus*; *Streptomyces herbaricolor*; *Streptomyces indigoferus*; *Streptomyces litmocidini*; *Streptomyces narbonensis*; *Streptomyces nashvillensis; Streptomyces noboritoensis*; *Streptomyces phaeopurpureus*; *Streptomyces purpeofuscus*; *Streptomyces showdoensis*; *Streptomyces tanashiensis*; *Streptomyces violaceorectus*; *Streptomyces zaomyceticus*; *Streptomyces aburaviensis*; *Streptomyces caeruleus*; *Streptomyces catenulae*; *Streptomyces chrysomallus* subsp. *fumigatus*; *Streptomyces xanthocidicus*; *Streptomyces achromogenes subsp. rubradiris*; *Streptomyces anandii*; *Streptomyces aurantiogriseus*; *Streptomyces bobili subsp. sporificans*; *Streptomyces cinerochromogenes*; *Streptomyces cirratus*; *Streptomyces collinus*; *Streptomyces eurythermus; Streptomyces galbus*; *Streptomyces galilaeus*; *Streptomyces griseoruber*; *Streptomyces griseosporeus*; *Streptomyces hygroscopicus* subsp. *ossamyceticus*; *Streptomyces kurssanovii*; *Streptomyces luteogriseus*; *Streptomyces massasporeus*; *Streptomyces mirabilis*; *Streptomyces multispiralis*; *Streptomyces naganishii*; *Streptomyces neyagawaensis*; *Streptomyces nojiriensis*; *Streptomyces olivochromogenes*; *Streptomyces phaeofaciens*; *Streptomyces pulveraceus*; *Streptomyces rameus*; *Streptomyces resistomycificus*; *Streptomyces rishiriensis*; *Streptomyces thermoviolaceus*; *Streptomyces violaceochromogenes*; *Streptomyces afghaniensis*; *Streptomyces arenae*; *Streptomyces attrocyaneus*; *Streptomyces chromofuscus*; *Streptomyces durhamensis*; *Streptomyces echinatus*; *Streptomyces filipinensis*; *Streptomyces fimbriatus*; *Streptomyces griseochromogenes*; *Streptomyces iakyrus*; *Streptomyces lucensis*; *Streptomyces malachitofuscus*; *Streptomyces malacbitorectus*; *Streptomyces pilosus*; *Streptomyces albidofuscus*; *Streptomyces albogriseolus*; *Streptomyces ambofaciens*; *Streptomyces anthocyanicus*; *Streptomyces antimycoticus*; *Streptomyces argenteolus*; *Streptomyces atratus*; *Streptomyces aureofaciens*; *Streptomyces avellaneus*; *Streptomyces caesius*; *Streptomyces carnosus*; *Streptomyces chibaensis*; *Streptomyces coelescens*; *Streptomyces coelicolor subsp. achrous*; *Streptomyces coelicolorsubsp. coelicofers*; *Streptomyces coelicolorsubsp. coelicolatus*; *Streptomyces coelicolor subsp. coelicovarians*; *Streptomyces corchorusii*; *Streptomyces cyanogenus*; *Streptomyces diastaticus* subsp. *ardesiacus*; *Streptomyces diastatochromogenes* subsp. *bracus*; *Streptomyces endus*; *Streptomyces erumpens*; *Streptomyces griseoaurantiacus*; *Streptomyces griseofuscus*; *Streptomyces griseolosuffuscus*; *Streptomyces griseoluteus*; *Streptomyces griseus subsp. difficilis*; *Streptomyces humidus*; *Streptomyces hygroscopicus*; *Streptomyces hygroscopicus* subsp. *angustmyceticus*; *Streptomyces hygroscopicus subsp. decoyicus*; *Streptomyces hygroscopius* subsp. *glebosus*; *Streptomyces libani*; *Streptomyces libani subsp. rufus*; *Streptomyces lividans*; *Streptomyces lusitanus*; *Streptomyces lydicus*; *Streptomyces melanosporofaciens*; *Streptomyces misionensis*; *Streptomyces murinus*; *Streptomyces mutabilis; Streptomyces nigrescens*; *Streptomyces nodosus*; *Streptomyces nogalater*; *Streptomyces olivaceiscleroticus*; *Streptomyces olivaceoviridis*; *Streptomyces olivaceus*; *Streptomyces parvullus*; *Streptomyces platensis*; *Streptomyces plicatus*; *Streptomyces poonensis*; *Streptomyces psammoticus*; *Streptomyces purpurogeneiscleroticus*; *Streptomyces recifenis; Streptomyces rochei; Streptomyces rokugoensis*; *Streptomyces roseodiastaticus*; *Streptomyces rutgersensis* subsp. *castelarense*; *Streptomyces sayamaensis*; *Streptomyces sendaiensis*; *Streptomyces sioyaensis*; *Streptomyces tendae*; *Streptomyces thermovulgaris*; *Streptomyces tricolor*; *Streptomyces tubercidicus*; *Streptomyces tumemacerans*; *Streptomyces vastus*; *Streptomyces violaceolatus*; *-Streptomyces violaceus-niger*; *- Streptomyces violaceus-ruber*; *Streptomyces viridifaciens*; *Streptomyces atroolivaceus*; *Streptomyces cyanocolor*; *Streptomyces graminofaciens*; *Streptomyces griseoplanus*; *Streptomyces albaduncus*; *Streptomyces albospiaus*; *Streptomyces albulus*; *Streptomyces althioticus*; *Streptomyces arabicus; Streptomyces atroolivaceus* subsp. *mutomycini*; *Streptomyces canus*; *Streptomyces chattanoogensis; Streptomyces chlorobiens*; *Streptomyces cuspidosporus*; *Streptomyces gancidicus*; *Streptomyces griseoflavus*; *Streptomyces griseoincarnatus*; *Streptomyces griseorubens*; *Streptomyces macrosporeus*; *Streptomyces malachiticus*; *Streptomyces matensis*; *Streptomyces noursei; Streptomyces olivoviridis*; *Streptomyces pseudogriseolus*; *Streptomyces rubiginosus*; *Streptomyces sparsogenes*; *Streptomyces viridiviolaceus*; *- Streptomyces virido-diastaticus*; *Streptomyces calvus*; *Streptomyces cyanoalbus*; *Streptomyces finlayi*; *Streptomyces flaveolus*; *Streptomyces geysiriensis*; *Streptomyces herbiferis*; *Streptomyces pactum; Streptomyces akitaensis*; *Streptomyces akiyoshiensis*; *Streptomyces alanosinicus*; *Streptomyces albidus* subsp. *invertens*; *Streptomyces albochromogenes*; *Streptomyces ansochromogenes*; *Streptomyces ansochromogenes* subsp. *pallens*; *Streptomyces avidinii; Streptomyces carcinomycicus*; *Streptomyces castaneglobisporus*; *Streptomyces castaneus*; *Streptomyces cyanoflavus*; *Streptomyces djakartensis*; *Streptomyces erythrochromogenes* subsp. *narutoensis*; *Streptomyces glomerochromogenes*; *Streptomyces grisinus*; *Streptomyces haranomachiensis*; *Streptomyces hygrostaticus*; *Streptomyces insulatus*; *Streptomyces inversochromogenes*; *Streptomyces kitazuwaensis*; *Streptomyces mariensis*; *Streptomyces minutiscleroticus*; *Streptomyces mitakaensis*; *Streptomyces nigrogriseolus*; *Streptomyces ogaensis*; *Streptomyces piedadensis*; *Streptomyces regensis*; *Streptomyces robefuscus*; *Streptomyces robeus*; *Streptomyces robustrus*; *Streptomyces roseogriseolus*; *Streptomyces roseogriseus*; *Streptomyces sahachiroi*; *Streptomyces senoensis*; *Streptomyces tanashiensis* subsp. *cephalomyceticus*; *Streptomyces thermonitrificans*; *Streptomyces thermoviolaceus* subsp. *apingens*; *Streptomyces viridoniger*; *Streptomyces werraensis*; *Streptomyces alboflavus*; *Streptomyces bacillaris*; *Streptomyces cavourensis*; *Streptomyces cyaneofuscatus*; *Streptomyces fulvissimus*; *Streptomyces griseobrunneus*; *Streptomyces michiganensis*; *Streptomyces tsusimaensis*; *Streptomyces xanthochromogenus*; *Streptomyces albidoflavus*; *Streptomyces alboviridis*; *Streptomyces anulatus*; *Streptomyces badius*; *Streptomyces californicus*; *Streptomyces canescens*; *Streptomyces celluloflavus*; *Streptomyces cellulosae*; *Streptomyces champavatii*; *Streptomyces chrysomallus*; *Streptomyces citreofluorescens; Streptomyces coelicolor, Streptomyces felleus*; *Streptomyces fimicarius*; *Streptomyces floridae*; *Streptomyces fluorescens*; *Streptomyces globisporus*; *Streptomyces globisporus* subsp. *caucasicus*; *Streptomyces globisporus* subsp. *flavofuscus*; *Streptomyces globisporus* subsp. *vulgaris*; *Streptomyces gougerotii; Streptomyces griseinus*; *Streptomyces griseoloalbus*; *Streptomyces griseus*; *Streptomyces griseus subsp. alpha; Streptomyces griseus* subsp. *cretosus*; *Streptomyces griseus* subsp. *solvifaciens*; *Streptomyces intermedius*; *Streptomyces kanamyceticus*; *Streptomyces levoris*; *Streptomyces limosus*; *Streptomyces lipmanii*; *Streptomyces microflavus*; *Streptomyces odorifer*; *Streptomyces parvus*; *Streptomyces* *pluricolorescens*; *Streptomyces pneumonicus*; *Streptomyces praecox*; *Streptomyces puniceus*; *Streptomyces raffinosus*; *Streptomyces rutgersensis*; *Streptomyces sampsonii; Streptomyces setonii; Streptomyces sindenensis*; *Streptomyces sulphureus*; *Streptomyces willmorei*; *Streptomyces hawaiiensis*; *Streptomyces albohelvatus*; *Streptomyces aurigineus*; *Streptomyces canarius*; *Streptomyces chryseus*; *Streptomyces flavidovirens*; *Streptomyces helvaticus*; *Streptomyces longisporoflavus*; *Streptomyces niveus*; *Streptomyces paucidisstaticus*; *Streptomyces spheroides*; *Streptomyces pimprina*; *Streptomyces capoamus*; *Streptomyces cinnabarinus*; *Streptomyces crystallinus*; *Streptomyces flavotricini*; *Streptomyces gobitricini*; *Streptomyces lincolnensis*; *Streptomyces melanogenes*; *Streptomyces phaeochromogenes*; *Streptomyces phaeochromogenes* subsp. *chloromyceticus*; *Streptomyces pseudovenezuelae*; *Streptomyces roseoviridis*; *Streptomyces spectabillis*; *Streptomyces subrutilus*; *Streptomyces umbrinus*; *Streptomyces venezuelae; Streptomyces xanthophaeus*; *Streptomyces aureomonopodiales*; *Streptomyces eufoliatus*; *Streptomyces filamentosus*; *Streptomyces prunicolor*; *Streptomyces roseofulvus*; *Streptomyces roseolus*; *Streptomyces roseoporus*; *Streptomyces rubiginosohelvolus*; *Streptomyces termitum; Streptomyces cinnamonensis*; *Streptomyces colombiensis*; *Streptomyces goshikiensis*; *Streptomyces katrae*; *Streptomyces lavendofoliae*; *Streptomyces lavendulae*; *Streptomyces lavendulae* subsp. *avireus*; *Streptomyces lavendulae* subsp. *brasilicus*; *Streptomyces lavendulae* subsp. *grasserius*; *Streptomyces lavendulcolor*; *Streptomyces luridus*; *Streptomyces orchidaceus*; *Streptomyces racemachromogenes*; *Streptomyces syringae*; *Streptomyces toxytricini*; *Streptomyces tuirus*; *Streptomyces vinaceus*; *Streptomyces virginiae*; *Streptomyces lateritus; Streptomyces flavovariabilis*; *Streptomyces janthinus*; *Streptomyces purpurascens*; *Streptomyces roseospinus*; *Streptomyces roseoviolaceus*; *Streptomyces violaceus*; *Streptomyces violaceus* subsp. *confinus*; *Streptomyces violaceus* subsp. *vicinus; Streptomyces violarus*; *Streptomyces violatus*; *Streptomyces yokosukanensis*; *Streptomyces albosporeus*; *Streptomyces aurantiacus*; *Streptomyces aureoverticillatus*; *Streptomyces aurini; Streptomyces cremeus*; *Streptomyces daghestanicus*; *Streptomyces fradiae*; *Streptomyces fragilis*; *Streptomyces fumanus*; *Streptomyces glomeroaurantiacus*; *Streptomyces griseoviridis*; *Streptomyces niveoruber, Streptomyces peucetius*; *Streptomyces phaeoviridis*; *Streptomyces roseiscieroticus*; *Streptomyces roseoflavus*; *Streptomyces roseolilacinus*; *-Streptomyces* *rubo-cyaneus*; *Streptomyces tauricus*; *-Streptomyces vinaceus-drappus*; *Streptomyces virocidus*; *Streptomyces erythraeus*; *Streptomyces luteofluorescens*; *Streptomyces erythrogriseus*; *Streptomyces garyphalus*; *Streptomyces lavendularectus*; *Streptomyces nagasakiensis*; *Streptomyces rubrolavendulae; Streptomyces cinnamonensis*; *Streptomyces ashchabadicus*; *Streptomyces polychromogenes*; *Streptomyces amakusaensis*; *Streptomyces caelestis*; *Streptomyces azureus*; *Streptomyces bellus*; *Streptomyces chartreusis*; *Streptomyces coeliatus*; *Streptomyces coerulatus*; *Streptomyces coerulatus subsp. amylolyticus*; *Streptomyces coeruleofuscus*; *Streptomyces coeruleorubidus*; *Streptomyces coerulescens*; *Streptomyces curacoi; Streptomyces cyaneus*; *Streptomyces cyanoglomerus*; *Streptomyces indigocolor*; *Streptomyces lanatus*; *Streptomyces lazureus*; *Streptomyces valynus*; *Streptomyces viridochromogenes*; *Streptomyces glaucescens*; *Streptomyces blensis*; *Streptomyces coerulatus subsp. anaseuli; Streptomyces coeruleoroseus*; *Streptomyces ipomoeae*; *Streptomyces spinosus*; *Streptomyces griseomycini*; *Streptomyces griseostramineus*; *Streptomyces prasinosporus*; *Streptomyces ghanaensis*; *Streptomyces hirsutus*; *Streptomyces prasinus*; *Streptomyces viridosporus*; *Streptomyces acrimycini*; *Streptomyces bambergiensis*; *Streptomyces prasinopilosus*; *Streptomyces horton; Streptomyces rectiviolaceus*; *Streptomyces lilacinofulvus*; *Streptomyces mauvecolor*; *Streptomyces violans*; *Streptomyces violascens*; *Streptoverticillium baldaccii*; *Streptoverticillium fervens*; *Streptoverticillium rubrochlorinum; Streptoverticillium biverticillatum*; *Streptoverticillium aureoversales; Streptoverticillium pentaticum; Streptoverticillium roseoverticillatum; Streptoverticillium rubroverticillatum*; *Streptoverticillium hiroshimense*; *Streptoverticillium salmonis*; *Streptoverticillium luteoverticillatum; Streptoverticillium olivoreticuli; Streptoverticillium waksmanii; Streptoverticillium griseocarneum*; *Streptoverticillium cinnamoneum*; *Streptoverticillium hachijoense*; *Streptoverticillium ardum*; *Streptoverticillium abikoense*; *Streptoverticillium albireticuli*; *Streptoverticillium eurocidicum; Streptoverticillium kishiwadense*; *Streptoverticillium mashuense*; *Streptoverticillium olivoverticillatum*; *Streptoverticillium orinoci; Streptoverticillium parvisporogenes*; *Streptoverticillium kentuckense*; *Streptoverticillium album; Streptoverticillium distallicum*; *Streptoverticillium ehimense*; *Streptoverticillium flavopersicum*; *Streptoverticillium griseoverticillatum*; *Streptoverticillium netropsis*; *Streptoverticillium* *rectiverticillatum*; *Streptoverticillium septatum; Streptoverticillium mobaraense*; *Streptoverticillium blastmyceticum; Streptoverticillium lavenduligriseum*; *Streptoverticillium lilacinum*; *Streptoverticillium kashmirense*; *Streptoverticillium thioluteum*; *Sporichthya polymorpha*; *Microellobosporia cinerea*; *Microellobosporia violacea*; *Microellobosporia flavea; Microellobosporia grisea*; *Micromonospora chalcea*; *Micromonospora halophytica*; *Micromonospora carbonacea*; *Micromonospora narashinoensis*; *Micromonospora melanosporea; Micromonospora echinospora*; *Micromonospora purpurea; Micromonospora purpureochromogenes*; *Micromonospora bicolor*; *Micromonospora coerulea*; *Micromonospora globosa*; *Micromonospora elongata*; *Micromonosora parva; Micromonospora gallica*; *Micromonospora acetoformici*; *Micromonospora propionici*; *Thermoactinomyces vulgaris*; *Thermoactinomyces sacchari*; *Actinobifida dichotomica; Actinobifida alba*; *Actinobifida chromogena*; *Thermomonspora curvata; Thermomonospora viridis*; *Microbispora rosea; Microbispora aerata; Microbispora amethystogenes*; *Microbispora bispora; Microbispora chromogenes*; *Microbispora diastatica; Microbispora parva; Microbispora thermodiastatica*; *Microbispora thermorosea*; *Micropolyspora brevicatena*; *Micropolyspora angiospora*; *Micropolyspora caesia; Micropolyspora faeni*; *Micropolyspora rectivirgula; Micropolyspora rubrobrunea; Micropolyspora thermovirida*; *Micropolyspora viridinigra*

A method for obtaining soluble ingredients derived from a microorganism, preferably a bacterium, is not particularly limited. Generally, they can be obtained by extraction from a microorganism. Conditions for the extraction are not particularly limited, and conditions available for those skilled in the art can be employed. A type of a solvent is not particularly limited. Preferably, any one of an alcohol, acetone, pyridine, hot water at 40°C or higher, which can easily be removed in the final step of washing, and a mixture thereof can be used.

In the aforementioned step (b), for example, one ampoule of a commercially available freeze-dried BCG preparation (Japan BCG Laboratory, content: 12 mg) is added with 1 ml of ethanol, sufficiently stirred, and then centrifuged at 12,000 x g (12,000 rpm) for 5 minutes by using a high speed microcentrifuge apparatus, and a resulting supernatant is collected, added to another ampoule of the freeze-dried BCG preparation, sufficiently stirred again, and centrifuged at 12,000 x g (12,000 rpm) for 5 minutes by using a high speed microcentrifuge apparatus, and further the resulting supernatant is collected to obtain an extract of soluble ingredients derived from a microorganism.

By adding the above extract to the fragments of the immobilized biological material produced in the step of (a), the soluble ingredients derived from a microorganism can be immobilized on the fragments. A means for the immobilization is not particularly limited. Any method available for those skilled in the art may be appropriately used. The term "immobilization" means a state that the soluble ingredients derived from a microorganism are temporarily or semipermanently bound to the surfaces of the fragments in a state that the soluble ingredients derived from a microorganism are not easily eliminated from the surfaces of the fragments, and the term should be construed in its broadest sense including formation of chemical bonds, physicochemical interactions and the like.

For example, the fixed biological tissue fragments are centrifuged at 12,000 x g (12,000 rpm) for 5 minutes by using a high speed microcentrifuge apparatus, added with 100 µl of ethanol extract of a bacterium on the basis of 10 µl of the fragments in terms of a packed volume, stirred, then added with 0.1-fold volume of sterilized ultrapure water, and stirred again. Then, the mixture was further added with 0.3-fold volume of sterilized ultrapure water, stirred again, and then added with sterilized ultrapure water up to a 100-fold volume of the original volume of the ethanol extract of the bacterium, i.e., 10 ml. By performing sufficient stirring in the above operation, the soluble ingredients derived from a microorganism are immobilized on the fragments of the solidified biological material. Then, the solidified biological material fragments are centrifuged and further washed with sterilized ultrapure water, thereby pure water-soluble ingredients are removed easily. Whilst the ethanol-soluble ingredients derived from a microorganism, that are not removed by this washing operation, remain in a state that they are bound to the fragments of the solidified biological material. The resulting product can be used as the adjuvant of the present invention. However, the aforementioned production method is given as an example, and a method for producing the adjuvant of the present invention is not limited to the aforementioned specific example. It will be readily understood by those skilled in the art that the aforementioned method can be optionally modified or altered, and the materials, conditions, and the like can be optionally chosen.

When the immunoadjuvant of the present invention is applied to a certain kind of animal, a tissue derived from an animal heterogenous to said animal can also be used as the solidified biological material in the aforementioned example. The adjuvant obtained as described above can be used as an insoluble immunoadjuvant accompanied by a rejection to a heterogenous animal tissue.

Moreover, a formalin-fixed tissue of an extracted tumor from a tumor patient may be used as the solidified biological material to produce an immunoadjuvant in which bacterium-derived soluble ingredients are immobilized. Such an immunoadjuvant can be used as a main ingredient of a tumor vaccine containing a human tumor antigen.

### Examples

The present invention will be explained more specifically by way of examples. However, the scope of the present invention is not limited to the following examples. The abbreviations used in the examples have the following meanings: CTL: cytotoxic T lymphocyte, FBS: fetal bovine serum, LPS: lipopolysaccharide, NK: natural killer, PBMC: peripheral blood monocyte, IL: interleukin, PBS(+):Dulbecco's phosphate buffered saline containing calcium and magnesium, and PBS(-): Dulbecco's phosphate buffered saline not containing calcium and magnesium.

### Example 1: Stimulation effect on human peripheral blood adhesive cells of an adjuvant in which BCG soluble ingredient is immobilized (formalin-fixed liver cancer tissue fragments stuck with BCG bacterium-derived ethanol-soluble ingredients)

### 1. Fragmentation and washing of formalin-fixed human liver cancer tissue

A resected human liver cancer tissue was fixed by immersion in a commercially available neutral formalin solution at room temperature for three days or more. This tissue was taken out, and made into small minces having a diameter of about 1 mm by using ophthalmic scissors, added with PBS(+) in a 3- to 10-fold volume on the basis of an original wet weight of the liver cancer used, and homogenized for 30 seconds by using a homogenizer (DIAX-600, Heidolph, generator shaft: TYPE 10F) with ice cooling. This homogenization was repeated several times with intervals of 30 seconds or more for ice cooling. The homogenate was taken into a 1.5-ml Eppendorf centrifuge tube in a volume of 1.2 ml, and centrifuged at 17,000 x g (15,000 rpm) for 5 minutes by using an Eppendorf high-speed microcentrifuge apparatus.

The precipitates were suspended in 70% alcohol, centrifuged to remove a supernatant, and then suspended again in PBS (+) of the original volume. The suspension was passed through a nylon mesh. The passed suspension was taken into a 1.5-ml Eppendorf centrifuge tube in a volume of 1.2 ml, and centrifuged at 15,000 rpm for 3 minutes by using a high-speed microcentrifuge apparatus, and the packed volume was measured. The measurement was performed by comparison with given amounts of water contained in 1.5-ml Eppendorf centrifugal tubes.

### 2. Preparation of ethanol-soluble ingredients derived from BCG bacterium cells

One ampoule of freeze-dried BCG vaccine (Japan BCG Laboratory, content: 12 mg) was autoclaved at 110°C for 5 minutes, added with 1 ml of ethanol, stirred for 6 hours or more, and then centrifuged at 12,000 x g (12,000 rpm) by using a high speed microcentrifuge apparatus for 5 minutes. The supernatant was collected, added to another ampoule of the freeze-dried BCG preparation, sufficiently stirred again, and centrifuged at 12,000 rpm for 5 minutes by using a high speed microcentrifuge apparatus, and the supernatant was collected as a solution of ethanol-soluble ingredients derived from the BCG bacterium cells (henceforth referred to as "BCG extract").

### 3. Preparation of BCG soluble ingredient-immobilized adjuvant

The PBS(+) suspension of the formalin-fixed human liver cancer tissue fragments washed in the aforementioned section 1 was diluted with PBS (-) to a density of approximately 1 million fragments/ml. This suspension in a volume of 1 ml was centrifuged at 12,000 x g (12,000 rpm) for 5 minutes, and the precipitates were added with 1 ml of 99.5% ethanol, and suspended in the ethanol. The suspension was centrifuged again at 12,000 rpm for 5 minutes to obtain precipitates. The precipitates were added with 0.1 ml of the BCG extract and stirred. Then, the suspension was slowly added with 0.01 ml of pure water with stirring, then further added with 0.03 ml and 0.1 ml of pure water, stirred for 1 hour each, added with 0.76 ml of pure water, stirred for 1 hour, added with 9 ml of pure water, and finally stirred overnight.

The above stirred suspension was washed three times with pure water by centrifugation. The precipitates were suspended in 1 ml of PBS(-), and centrifuged again to obtain precipitates, and the precipitates were suspended to 1 ml of the RPMI culture medium containing 20% FBS. This suspension was used as the culture medium for stimulating the cells containing BCG soluble ingredient-immobilized adjuvant. In addition, as shown in Table 1, culture media for stimulating cells were prepared which contained various kinds of additives, such as a culture medium for stimulating cells which contained formalin-fixed liver cancer tissue fragments in the same amount as that of the BCG soluble ingredient-insolubilized adjuvant.

### 4. Preparation of BCG soluble ingredient-coated sheet fragment

As a control for the immobilized adjuvant prepared by coating the formalin-fixed human liver cancer tissue fragments with the BCG extract, a sheet fragment was prepared by dropping 2 µl of the BCG extract onto a foaming plastic sheet (packing material for a lid of a reagent bottle of sodium hydrogencarbonate (Wako Pure Chemical Industries)) of a 3 mm square and air-drying the extract. This BCG soluble ingredient-coated sheet fragment can float on a culture medium, and even if the fragment is added to a cell culture medium together with the fixed human liver cancer tissue fragments, the two fragments immediately dissociate from each other, and the fixed human liver cancer tissue fragment is not coated with the ingredients in the BCG extract.

### 5. Preparation of LPS as a positive control

A lipopolysaccharide (hereinafter referred to as "LPS," Sigma Aldrich Japan, Inc., Tokyo), which is known to stimulate human peripheral blood adhesive cells to induce release of GM-CSF, was dissolved in the RPMI culture medium containing 20% FBS at a final concentration of 10 ng/ml.

### 6. Preparation of human peripheral blood adhesive cells

Peripheral blood of healthy human subject was collected in a conventional manner as a heparin-treated blood. The heparin-treated blood in a volume of 15 ml was diluted with the same volume of PBS(-), slowly added to a Lymphoprep tube (NYCOMED PHARMA, Norway) so that the heparin-treated blood was placed on a net in the tube, and centrifuged at 800 x g for 20 minutes. The cloudy layer rich in leucocytes close above the net was collected, and washed with PBS(-). The washing was performed by centrifugation according the operation manual by the manufacturer of Lymphoprep.

To a 96-well culture plate, 40 µl per well of RPMI culture medium containing 20% FBS was added, and appropriately incubated beforehand. A cell suspension, which was obtained by diluting the cells obtained from the aforementioned leukocyte fraction with the RPMI culture medium containing 20% FBS to a density of 5 million cells/ml, was inoculated in a volume of 200 µl per well. After incubation for 1 hour, the adhered cells were washed twice with the RPMI culture medium containing 20% FBS, which was warmed to 37°C beforehand. When the cells were observed under a microscope, large numbers of monocytes were observed among the adhesive cells.

### 7. Stimulation of human peripheral blood adhesive cells with BCG soluble ingredient-immobilized adjuvant

The washed adhesive cells were added with 200 µl per well of the culture medium for stimulating cells, and then cultured for 24 hours. The supernatant was collected and centrifuged at 12,000 x g (12,000 rpm) for 5 minutes to obtain a supernatant. This supernatant was stored at -80°C. One piece of BCG soluble ingredient-coated sheet fragment was floated on the culture medium per each well with the BCG soluble ingredient-coating surface being on the down side.

### 8. Method for measuring adhesive cell-stimulating effect

By using a kit for measuring human GM-CSF on the basis of the ELISA method (Amersham, England), a GM-CSF content in the stored supernatant mentioned in the above section was quantified according to the measurement manual supplied by the manufacturer of the kit.

The results are shown in Table 1. Each average measured value (described as a concentration in the culture supernatant in terms of pg/ml) was obtained from values obtained for two of separate wells.

It was found that the BCG soluble ingredient-immobilized adjuvant had a human peripheral blood adhesive cell-stimulating effect in a degree comparable to that of LPS (final concentration: 10 ng/ml) used as the positive control. When the formalin-fixed liver cancer tissue fragments derived from the same individual as the control was used, on which the BCG soluble ingredients were not immobilized, only a stimulating effect corresponding to 1/6 or less of GM-CSF concentration was obtained. Even when these fragments were added together with the BCG soluble ingredient-coated sheet fragment, no significant stimulating effect comparable to that of the BCG soluble ingredient-immobilized adjuvant was obtainable. Therefore, it can be understood that the immobilization of BCG soluble ingredients on the formalin-fixed liver cancer tissue fragments is important.

**Table 1**

| Ingredient added to culture medium for stimulating cells | GM-CSF concentration in culture supernatant (pg/ml) |
|---|---|
| 1. Control (culture medium alone) | 5.9 |
| 2. Formalin-fixed liver cancer tissue fragment | 13.4 |
| 3. BCG soluble ingredient-immobilized adjuvant | 85.8 |
| 4. BCG soluble ingredient-coated sheet fragment | 11.5 |
| 5. Formalin-fixed liver cancer tissue fragments + BCG soluble ingredient-coated sheet fragment | 7.3 |
| 6. BCG soluble ingredient-immobilized adjuvant + BCG soluble ingredient-coated sheet fragment | 71.8 |
| 7. LPS for positive control | 71.1 |

### Example 2: Comparison of human peripheral blood adhesive cell-stimulating effects of BCG soluble ingredients and BCG soluble ingredient-immobilized adjuvant

According to the methods of Example 1, fragmentation and washing of a formalin-fixed human liver cancer tissue, preparation of BCG cell-derived ethanol-soluble ingredients, preparation of BCG soluble ingredient-immobilized adjuvant, preparation of LPS for positive control, preparation of human peripheral blood adhesive cells, stimulation of human peripheral blood adhesive cells with the BCG soluble ingredient-immobilized adjuvant, and measurement of adhesive cell-stimulating effect were performed, provided that 2 µl per well of the BCG extract was added to the 96-well culture plate instead of floating the BCG soluble ingredient-coated sheet fragment on the culture medium. For comparison, 2 µl per well of ethanol was added to the other wells.

The results are shown in Table 2. Each average measured value (described as a concentration in the culture supernatant in terms of pg/ml) was calculated from values obtained from four independent wells, except for the control (culture medium alone) and the wells added with ethanol. As for the human peripheral blood adhesive cell-stimulating effect compared in terms of concentrations of produced GM-CSF, the well, in which BCG soluble ingredients were not immobilized beforehand and the formalin-fixed liver cancer tissue fragments and the BCG extract were separately added, gave less effect than the well in which the BCG soluble ingredient-immobilized adjuvant obtained by integrating the BCG soluble ingredients and the fixed liver cancer tissue fragments by the advanced immobilization. This result indicates that the BCG soluble ingredients that are still soluble have a weak GM-CSF production-stimulating effect (i.e., a weak adjuvant effect), and the potent cell-stimulating effect was dependent on the immobilization of the BCG soluble ingredients. From the results of Example 1, it is considered that immobilization of the BCG soluble ingredients on the fixed liver cancer tissue fragments is important, and the BCG soluble ingredients are also taken into the cells because the formalin-fixed liver cancer tissue fragments are phagocytized by the cells. The BCG soluble ingredients can stimulate cells from the inside of the cells even at a trace amount, and therefore, they exhibit extremely efficient stimulating effect. It is considered that the immobilized adjuvant which activates the cells as described above provides, as a course of event, an effect of further stimulating the intracellular disposition of the formalin-fixed liver cancer tissue fragments taken into the cells.

**Table 2**

| Ingredient added to culture medium for stimulating cells | GM-CSF concentration in culture supernatant (pg/ml) |
|---|---|
| 1. Control (culture medium alone) | 7.8* |
| 2. Control (culture medium alone) + ethanol | 8.2* |
| 3. Formalin-fixed liver cancer tissue fragment | 8.1 ± 0.1** |
| 4. Formalin-fixed liver cancer tissue fragment + ethanol | 8.1 ± 0.4** |
| 5. BCG soluble ingredient-immobilized adjuvant | 88.8 ± 29.2** |
| 6. BCG soluble ingredient-immobilized adjuvant + ethanol | 68.0 ± 19.3** |
| 7. Formalin-fixed liver cancer tissue fragment + BCG extract | 8.4 ± 0.1** |
| 8. BCG extract | 8.3 ± 0.3** |
| 9. LPS for positive control | 196 ± 28.0** |
| 10. LPS for positive control + ethanol | 141 ± 13.4** |

| | |
|---|---|
| *: Observed value of one well, | |
| **: average value for four wells ± standard deviation | |

### Industrial Applicability

The immunoadjuvant of the present invention has a potent cell-stimulating effect, and can be used as an immunoadjuvant which is highly safe for living bodies. By using a formalin-fixed tumor tissue as a fixed biological tissue, complex and diverse tumor antigens and immunoadjuvants can be efficiently taken into antigen-presenting cells as an integrated form. Therefore, effective tumor immunoreactions can be induced, and thus effective cancer treatments are achievable.

## Claims

1. An immunoadjuvant comprising a fragment, wherein said fragment is prepared from a solidified material selected from the group consisting of a tissue and a cell of an animal including a human and an ingredient thereof, and from which fragment a soluble ingredient is removed by washing with an organic solvent and/or hot water, and wherein a soluble ingredient derived from a microorganism is immobilized to said fragment.

2. The immunoadjuvant according to claim 1, wherein the human tissue or the cell is a tumor tissue and/or a tumor cell.

3. The immunoadjuvant according to claim 1 or 2, wherein the solidified material selected from the group consisting of a tissue and a cell of an animal including a human and an ingredient thereof is a formalin-fixed tissue and/or a formalin-fixed cell.

4. The immunoadjuvant according to any one of claims 1 to 3, wherein the microorganism is a bacterium.

5. The immunoadjuvant according to any one of claims 1 to 4, wherein the soluble ingredient derived from a microorganism consists of at least one kind of an extract selected from the group consisting of an alcohol extract, an acetone extract, a pyridine extract, and a hot water extract.

6. A method for producing an immunoadjuvant, which comprises the steps of:
(a) washing a fragment prepared from a solidified material selected from the group consisting of a tissue and a cell of an animal including a human and an ingredient thereof with an organic solvent and/or hot water to remove a soluble ingredient; and
(b) immobilizing a soluble ingredient derived from a microorganism on the fragment obtained in the step (a).

7. A tumor vaccine which comprises, as an active ingredient, an immunoadjuvant comprising a fragment, wherein said fragment is prepared from a solidified material selected from the group consisting of a tissue and a cell of an animal including a human and an ingredient thereof, and from which fragment a soluble ingredient is removed by washing with an organic solvent and/or hot water, and wherein a soluble ingredient derived from a microorganism is immobilized to said fragment.

8. A method for therapeutic treatment of a tumor, which comprises the step of administering the tumor vaccine according to claim 7 to a patient from whom the solidified material is derived.
